# EUROPEAN PATENT SPECIFICATION

(11) **EP 2 231 201 B1**
(45) Date of publication and mention of the grant of the patent: **14.02.2018**
(21) Application number: 08865548.5
(22) Date of filing: 12.12.2008
(51) Int. Cl.: A61K 51/08, C07B 59/00, A61K 101/02

(54) **SELECTIVE RADIOLABELING OF BIOMOLECULES**
SELEKTIVE RADIOAKTIVE MARKIERUNG VON BIOMOLEKÜLEN
RADIOMARQUAGE SÉLECTIF DE BIOMOLÉCULES

(30) Priority: 20.12.2007 US 961108
(43) Date of publication of application: 29.09.2010
(60) Divisional application: 13154289.6
(73) Proprietor: General Electric Company, Schenectady, NY 12345 (US); GE Healthcare Limited, Little Chalfont Buckinghamshire HP7 9NA (GB)
(72) Inventor: PADILLA DE JESUS, Omayra Liz, Clifton Park New Jersey 12065 (US); KOVACS, Ernest William, Albany New York 12210 (US); GLASER, Matthias Eberhard, London W12 0NN (GB); ARSTAD, Erik, London NW1 2BU (GB); SYUD, Faisal Ahmed, Clifton Oaks New York 12065 (US)
(74) Representative: Aldenbäck, Ulla Christina
(86) International application number: PCT/EP2008/067413
(87) International publication number: WO 2009/080561

(56) References cited:
- WO-A-2006/067376
- LI ZI-BO ET AL: "Click chemistry for (18)F-labeling of RGD peptides and microPET imaging of tumor integrin alphavbeta3 expression" BIOCONJUGATE CHEMISTRY, ACS, WASHINGTON, DC, US, vol. 18, no. 6, 1 November 2007 (2007-11-01), pages 1987-1994, XP002470093 ISSN: 1043-1802
- MARIK J ET AL: "Click for PET: rapid preparation of [<18>F]fluoropeptides using Cu<I> catalyzed 1,3-dipolar cycloaddition" TETRAHEDRON LETTERS, ELSEVIER, AMSTERDAM, vol. 47, no. 37, 11 September 2006 (2006-09-11), pages 6681-6684, XP025004843 ISSN: 0040-4039 [retrieved on 2006-09-11]
- GREG T. HERMANSON: "Bioconjugate Techniques" 1996, ACADEMIC PRESS , SAN DIEGO, US , XP002523340 pages 146-152, section "Thiol-Reactive Chemical Reactions"
- CAI W ET AL: "A thiol-reactive <18>F-labeling agent, N-[2-(4- <18>F-fluorobenzamido) ethyl]maleimide, and synthesis of RGD peptide-based tracer for PET imaging of [alpha]v[beta]3 integrin expression" JOURNAL OF NUCLEAR MEDICINE 20060701 US, vol. 47, no. 7, 1 July 2006 (2006-07-01), pages 1172-1180, XP002470156 ISSN: 0161-5505
- RAMENDA T ET AL: "Synthesis of <18>F-labeled neurotensin(8-13) via copper-mediated 1,3-dipolar [3+2]cycloaddition reaction", LETTERS IN DRUG DESIGN AND DISCOVERY, BENTHAM SCIENCE PUBLISHERS, US, [Online] vol. 4, no. 4, 1 June 2007 (2007-06-01), pages 279-285, XP008105151, ISSN: 1570-1808, DOI: DOI:10.2174/157018007784619998 Retrieved from the Internet: URL:http://www.bentham.org/lddd/contabs/ld dd4-4.htm#7>

## Description

### BACKGROUND

Methods to introduce fluorine atoms, particularly radioactive fluorine atoms onto biomolecules comprising amino acid residues are of considerable interest. However, since radioactive fluorine atoms, such as ¹⁸F, have a relatively short lifetime of about 110 minutes, time-efficient methods are required to introduce radio fluorine onto biomolecules.

There is a continuing need for efficient and site-specific methods for introducing fluorine atom(s) including radioactive fluorine atom(s) onto biomolecules.

### BRIEF DESCRIPTION

The invention is defined by the appended claims. Any information falling outside the scope of the claims is for information only.

In one aspect, methods for introducing fluorine atom onto a biomolecule, such as polypeptide are disclosed. The methods may comprise: (i) providing a linker comprising a thiol-reactive terminus and an azido/alkyne-reactive terminus; (ii) reacting the thiol-reactive terminus of the linker with a biomolecule including at least one thiol group or a reactive derivative thereof; and (iii) subsequently reacting the azido/alkyne-reactive terminus of the linker with a fluorine-substituted azide or alkyne respectively.

In still another aspect, bioconjugates comprising structural units derived from (i) a biomolecule comprising at least one thiol group; and (ii) a linker; are provided. The linker may be prepared by a method comprising reacting an amine compound comprising an azido or alkyne-reactive functional group with a carboxylic acid or an activated ester comprising a thiol-reactive functional group.

In another aspect, the compositions of linkers are provided.

### FIGURES

These aspects, and advantages of the present invention will become better understood when the following detailed description is read with reference to the accompanying drawings in which like characters represent like parts throughout the drawings, wherein
FIG. 1 shows HPLC analysis of a reaction mixture of the non-optimized system showing ¹⁸F click labeled Anti-Her2 affibody (27) (a, radioactivity channel; b, UV channel at 280 nm).
FIG. 2 shows HPLC analysis of a reaction mixture containing 2-[¹⁸F]Fluoroethylazide (11) using gradient I, UV at 216 nm (a, radioactivity channel, 2-[¹⁸F]Fluoroethylazide (11) at 3:51 min; b, UV channel). The distilled product shows only the presence of 2-[¹⁸F]Fluoroethylazide (11) (c, radioactivity channel; d, UV channel).
FIG. 3 shows the HPLC plot of radiochemical yield of triazole product (17) formed at different alkyne (16) concentrations and temperatures using model compound (16) and 2-[18F]Fluoroethylazide (11).
FIG. 4 shows the MALDI-TOF MS data of an alkynylated affibody (22).
FIG. 5 shows the SDS-PAGE protein gel of a click conjugate between alkynylated affibody (22) and Cy5 labeled Azido PEG (25a). Fluorescence emission of Cy5 dye is observed at expected molecular weight of conjugate product (26) when in the presence of Cu^{I} source and reducing reagent. No presence of conjugated product is observed in the absence of any of these in the control experiments.

### DETAILED DESCRIPTION

To more clearly and concisely describe and point out the subject matter of the claimed invention, the following definitions are provided for specific terms that are used in the following description and the claims appended hereto. The singular forms "a", "an", and "the" include plural referents unless the context clearly dictates otherwise.

As used herein, the term "activating group" refers to any group that makes a carbonyl group more reactive towards nucleophiles, for example, N-hydroxysuccinimide, sulfo-N-hydroxysuccinimide, acid chloride, and urea intermediates.

As used herein, the term "azido-reactive terminus" and/or "azido-reactive functional group" refers to any functional group that can react with an azide functional group. Some examples of azido-reactive functional groups include, but are not limited to, alkynes, allenes and phosphines.

As used herein, the term "alkyne-reactive terminus" and/or "alkyne-reactive functional group" refers to any functional group that can react with an alkyne functional group. Examples of an alkyne-reactive functional group includes, but it is not limited to, azides, which can react in the presence of a source of Cu^{I}, including but not limited to, Cu⁰, Cu^{I}, Cu^{II}, and a reducing reagent such as sodium ascorbate.

As used herein, the term "aliphatic radical" or "aliphatic group" generally refers to an array of carbon atoms that is not cyclic and has a point of attachment that is an sp³ carbon atom. The array of carbon atoms may further comprise any combination of sp³, sp², or sp hybridized carbon atoms. Further, the array of carbon atoms may be monovalent, divalent, or trivalent. Examples of alkyl groups include methyl, ethyl, n-propyl, isopropyl, n-butyl, tert-butyl, isooctyl, benzyl, cyclohexylmethyl, and phenethyl, 1',1'-dimethylbenzyl.

As used herein, the terms "aromatic radical" or/and "aromatic group" refers to a cyclic array of sp² hybridized carbon atoms and conjugated carbon-carbon double bonds, and has a point of attachment at an aromatic sp² hybridized carbon atom that forms part of the cyclic array of carbon atoms. The aromatic group or radical may have from one to the maximum permissible number of substituents. Examples of aryl groups include phenyl, substituted phenyl, tolyl, substituted tolyl, xylyl, mesityl, chlorophenyl, naphthyl, furyl, thienyl, and pyrrolyl.

As used herein, the term "cycloalkyl radical" or a "cycloalkyl group" refers to a cyclic array of sp³ hybridized carbon atoms, and has a point of attachment at an sp³ hybridized carbon atom that forms part of the cyclic array of carbon atoms. The array of carbon atoms may further comprise any combination of sp³, sp², or sp hybridized carbon atoms. Further, the cyclic array of carbon atoms may be substituted with one to the maximum permissible number of substituents. Furthermore, the array of cyclic atoms may comprise heteroatoms, such as O, N, or S. Examples of cycloalkyl groups include cyclohexyl, methylcyclohexyl, trimethylcyclohexyl, phenylcyclohexyl, tetrahydropyranyl, 4-thiacyclohexyl, and cyclooctyl.

The term "a disulfide group capable of a thiol exchange reaction with a thiol group" refers to groups that may react with a thiol group of a biomolecule. Thus, a disulfide may be regarded as a thiol-reactive group. Pyridyl disulfide is an example of such a disulfide.

As used herein, the term "fluorine-substituted azide" denotes an azide-containing compound having at least one fluorine substituent. Further, the fluorine substituent may be of any isotopic variety, such as for example, ¹⁸F and ¹⁹F. In one embodiment, the fluorine substituent is ¹⁸F. The azide may be an aliphatic azide, a cycloaliphatic azide, or an aromatic azide. Furthermore, the cycloaliphatic azides and aromatic azides may have monocyclic, bicyclic, or polycyclic structures.

As used herein, the term "fluorine-substituted alkyne" denotes an alkyne-containing compound having at least one fluorine substituent. Further, the fluorine substituent may be of any isotopic variety, such as for example, ¹⁸F and ¹⁹F. In one embodiment, the fluorine substituent is ¹⁸F. The alkyne may be an aliphatic alkyne, a cycloaliphatic alkyne, or an aromatic alkyne. Furthermore, the cycloaliphatic alkynes and aromatic alkynes may have monocyclic, bicyclic, or polycyclic structures.

As used herein, the terms "protein", "peptide" and "polypeptide" are used herein to describe any chain of amino acids, regardless of length or post-translational modification such as glycosylation or phosphorylation. Thus, the terms may be used interchangeably herein to refer to a polymer of amino acid residues. The terms also apply to amino acid polymers in which one or more amino acid residue is an artificial chemical mimetic of a corresponding naturally occurring amino acid. Thus, the term "polypeptide" includes full-length, naturally occurring proteins as well as recombinantly or synthetically produced polypeptides that correspond to a full-length naturally occurring protein or to particular domains or portions of a naturally occurring protein.

The terms "radical" and "group", as applied to the terms "alkyl", "aliphatic", "cycloaliphatic", and "aromatic" are used interchangeably herein.

As used herein the term "thiol-reactive terminus" and/or "thiol-reactive functional group" refers to a functional group that may react with a thiol group or a mercaptan group (i.e., -SH group). Examples of thiol-reactive functional groups include, but are not limited to a maleimido group, a haloaliphatic group, a haloaromatic group, a halocycloaliphatic group, a (haloacetyl)alkyl group, a (haloacetyl)cycloalkyl group, a (haloacetyl)aryl group, an α, (β-unsaturated sulfone group, a vinyl sulfone group, an α, β-unsaturated carbonyl group, an epoxy group, an aziridine group, and a disulfide group capable of a thiol exchange reaction with a thiol group.

Suitable maleimido groups include the parent (unsubstituted) group as well as derivatives comprising aliphatic, cycloaliphatic or aromatic groups as substituents. Suitable α, β-unsaturated carbonyl groups include those comprising an acryloyl group. Suitable α, β-unsaturated carbonyl groups include α, β-unsaturated esters and α, β-unsaturated sulfones. Vinyl sulfone group is a specific example of an α, β-unsaturated sulfone group.

Unless otherwise indicated, all numbers expressing quantities of ingredients, properties such as molecular weight, reaction conditions, so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Aspects of the invention relate to a method for introducing a fluorine atom onto a biomolecule. The method includes the steps of
(i) providing a linker including a thiol-reactive terminus and an azido or an alkyne-reactive terminus;
(ii) reacting the thiol-reactive terminus of the linker with the biomolecule including at least one thiol group or a reactive derivative thereof; and
(iii) subsequently reacting the azido or the alkyne -reactive terminus of the linker with a fluorine-substituted azide or alkyne group respectively.

Another aspect of the invention is to provide a bioconjugate composition comprising structural units derived from a biomolecule comprising at least one thiol group and a linker. The linker may be prepared by a method comprising reacting an amine compound comprising an azido or alkyne-reactive functional group with a carboxylic acid or an activated ester comprising a thiol-reactive functional group. In another aspect the linker may be prepared by reacting an amine compound comprising a thiol-reactive functional group with a carboxylic acid or an activated ester compound comprising an azido or alkyne-reactive functional group.

### Biomolecules

As used herein, the term "biomolecules" refers to naturally occurring or engineered molecules that comprise at least one thiol group (also sometimes referred to as "mercapto" group) or a reactive derivative thereof for reaction with the linker. The thiol group may either occur naturally in the biomolecule or may be chemically introduced or engineered using standard biological methods or suitable art recognized methods. In some embodiments, the biomolecules specifically exclude having fewer than 50 amino acid residues.

Such biomolecules may either have one or more SH groups in their natural state, or they may be engineered, for example using standard molecular biology techniques. Examples of biomolecules having a SH group naturally include those linked to one or more cysteine amino acids. By the term "reactive derivative thereof" is meant a derivative of the SH group which is activated so as to generate the free thiol group for reaction with the linker compound.

Examples of biomolecules that comprise either one or more thiol groups naturally or chemically engineered thiol groups include peptides, polypeptides, vector, lipids, polysaccharides, glycosaminoglycans and modified versions thereof, glycolipids, glycoproteins, synthetic polymers, cell response modifiers, (e.g., growth factors, chemotactic factors, or cytokines), enzymes, receptors, neurotransmitters, hormones, cytokine, vaccines, haptens, toxins, interferons, and ribozymes. The disclosed methods may also be applied to molecules that do not contain a thiol group, but are conjugated to a molecule that does contain a thiol group. Further examples of biomolecules include proteins, protein fragments, protein variants, scaffold-based proteins, engineered proteins, nucleotides and related molecules, nucleic acids, oligo-DNA or oligo-RNA peptide conjugates, antibodies such as polyclonal and monoclonal antibodies, and antibody-based fragments. Thus, the disclosed methods may be used to fluorinate nucleic acids including deoxyribonucleic acids (e.g., oligodeoxynucleotides, nucleic acid probes, plasmids), ribonucleic acids (e.g., siRNA) associated with a thiol-containing molecule such as a polypeptide that has a thiol group.

The biomolecules may be any naturally occurring or engineered biomolecules having at least one thiol group. In all embodiments, the biomolecule may include naturally occurring and/or non-natural amino acid residues. In another embodiment, the biomolecule comprising the at least one thiol group comprises a cysteine residue or an unnatural group. By the term "cysteine residue" is meant the structural fragment other than the thiol group that results after cysteine is included as a part of the biomolecule chain. In still another embodiment, the biomolecule for reaction with the linker is one that has an engineered cysteine residue, which means that a suitable precursor biomolecule may be chemically modified to generate the desired biomolecule having the thiol group and the cysteine residue.

In some cases, a biomolecule may be treated with a reducing agent to generate a reactive thiol group. For example, a biomolecule having a disulfide linkage may be reduced with a suitable reducing agent to produce two equivalents of a biomolecule having a thiol group. Examples of useful reducing agents include 2-mercaptoethanol, 2-mercaptoethanolamine, dithiothreitol (DTT), and tris-(2-carboxyethyl)phosphine (TCEP).

### Linkers :

As used herein, the term "linker" refers to a bi-functional compound comprising a thiol-reactive terminus or a protected derivative thereof, and an azido or alkyne-reactive terminus or a protected derivative thereof. The linkers may be used to attach a thiol-containing compound at one end via the thiol-reactive terminus, and for attachment to azide or alkyne, especially fluorine-substituted azide or alkyne at the other end via the azido or alkyne-reactive terminus respectively. As used herein, the term "Mal-alkyne bifunctional linker" refers to a linker having a maleimido group at a first terminal and an alkyne group at a second terminal, such as N-(but-3-ynyl)-3-(2,5-dioxo-2,5-dihydro-1H-pyrrol-1-yl)propanamide linker (1) and "Mal-azide bifunctional linker" refers to a linker having a maleimido group at a first terminal and an azide group at a second terminal, such as compound (2). The linkers of the invention are shown in structures (1)-(6).

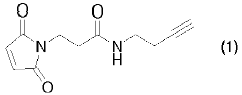

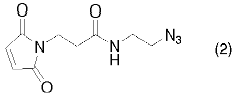

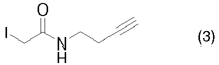

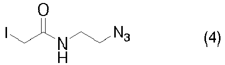

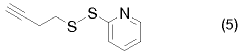

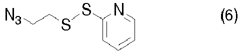

The linkers may be used to attach a thiol-containing compound at one end via the thiol-reactive terminus, and/or to attach azide or alkyne (e.g., fluorine-substituted azide or alkyne) at the other end via the azido or alkyne -reactive terminus respectively.

### Linker Preparation

The linker may be prepared by any method that makes both the thiol-reactive functional group and the azido or alkyne-reactive functional group accessible for reaction with (i) the biomolecule having at least one thiol group, and (ii) the fluorine-substituted azide or alkyne, respectively. In one embodiment, the linker is prepared by reacting an amine compound comprising an azido or alkyne-reactive functional group with a carboxylic acid or an activated ester comprising a thiol-reactive functional group. Any amine compound having an azido or alkyne reactive functional group may be used. In an embodiment, the amine compound comprises a structure (7),

G-J-NHR¹ (7)

wherein G is an azido or alkyne-reactive functional group, J is a linking unit, and R¹ is H, an aliphatic radical, an aromatic radical, or a cycloaliphatic radical. The nature of the divalent linking unit J is designed to minimize steric hindrance, which could adversely affect the reactivity of the thiol-reactive and the azido or alkyne-reactive functional groups.

In other embodiments, the linker may be prepared by reacting an amine compound comprising a thiol-reactive functional group with a carboxylic acid or an activated ester compound comprising an azido or alkyne-reactive functional group.

The carboxylic acid or activated ester comprises a structure (8),

L-M-COR² (8)

wherein L comprises a thiol-reactive functional group, or a protected derivative thereof; M is a divalent linking unit, and R² is OH or an activating group. The activating group R² facilitates the reaction of the amine compound having the azido or alkyne-reactive functional group with the carbonyl carbon atom of structure (8). The linkers of the invention having structures (1)-(6) have the following common formula:

L-M-CO- NH(R¹)-J-G,

where L is a thiol-reactive functional group;
M and J are the bifunctional units/linking units;
R¹ is H, an aliphatic radical, an aromatic radical, or a cycloaliphatic radical.; and
G is an azido or alkyne-reactive functional group
Exemplary synthetic approaches for preparing the linker having structure (1) and (2) are shown in Reaction Schemes 1 and 2.

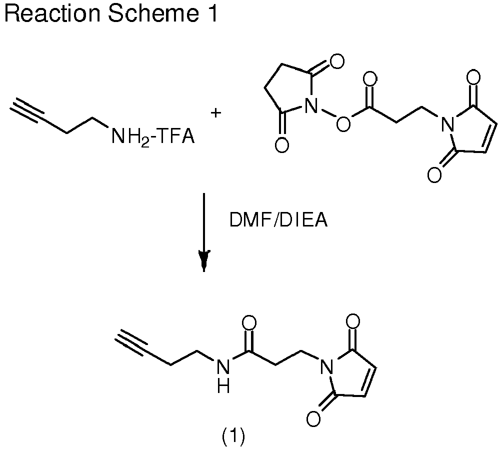

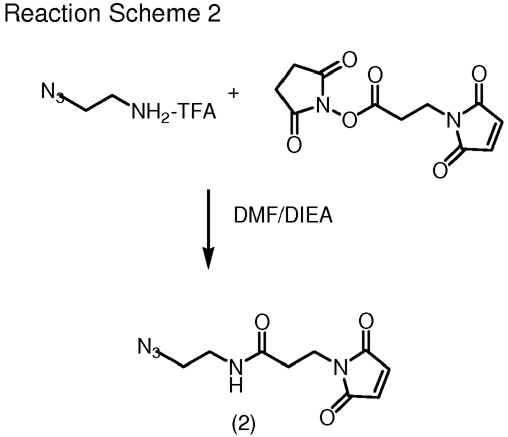

In Reaction Schemes 1 and 2, DMF stands for Dimethyl Formamide and DIEA stands for Di-isopropyl ethyl amine. Some examples of linkers that may be prepared using these methods are shown above in structures (1) to (6).

### Bioconjugates

The products resulting from the reaction of the linker with the biomolecule having at least one thiol group is termed as a bioconjugate. Thus in an embodiment, the bioconjugate comprises structural units derived from: (i) a biomolecule comprising at least one thiol group; and (ii) a linker; where the linker is prepared by a method comprising reacting an amine compound comprising an azido or alkyne-reactive functional group with a carboxylic acid or an activated ester comprising an thiol-reactive functional group. In another embodiment, the bioconjugate comprises structural units derived from: (i) a biomolecule comprising at least one thiol group; and (ii) a linker; where the linker is prepared by a method comprising reacting an amine compound comprising a thiol-reactive functional group with a carboxylic acid or an activated ester comprising an azido or alkyne-reactive functional group. In another aspect the linker may be prepared by reacting an amine compound comprising a thiol-reactive functional group with a carboxylic acid or an activated ester compound comprising an azido or alkyne-reactive functional group.

### Methods for Fluorinating Bioconjugates

The methods described herein enable the preparation of fluorine-labeled bioconjugates, more particularly radiofluorine (e.g., ¹⁸F) labeled bioconjugates. One of the advantages of these methods is that the linker may be attached selectively to a biomolecule such as a biomolecule under non-radioactive conditions in which the thiol group of the biomolecule may be reacted selectively with the thiol-reactive functional group of the linker, and the resulting bioconjugate may be purified prior to reaction with an ¹⁸F or a normal fluorine-substituted azide or alkyne. Another advantage is that the radiofluorine label may be added selectively in a final step, eliminating the need for time consuming additional purification steps before the preparation of the final bioconjugate, especially at tracer levels.

The methods described herein for introducing fluorine onto a bioconjugate may be used to generate fluorinated bioconjugate of any length. Thus, in some embodiments the biomolecule of the bioconjugate comprises at least 50 amino acid residues or at least 100 amino acid residues.

In one aspect, methods for introducing one or more fluorine atom(s) onto a biomolecule are disclosed. The methods may comprise: (i) providing a linker comprising a thiol-reactive terminus and an azido or alkyne-reactive terminus; (ii) reacting the thiol-reactive terminus of the linker with a biomolecule comprising at least one thiol group or a reactive derivative thereof; and (iii) subsequently reacting the azido or alkyne-reactive terminus of the linker with a fluorine-substituted azide or alkyne respectively.

More specifically, the methods described herein may be employed to introduce one or more fluorine atoms onto a biomolecule using Mal-alkyne bifunctional linker such as N-(but-3-ynyl)-3-(2,5-dioxo-2,5-dihydro-1H-pyrrol-1-yl)propanamide (1) as the linker. Such methods comprise: (i) reacting the thiol-reactive group of N-(but-3-ynyl)-3-(2,5-dioxo-2,5-dihydro-1H-pyrrol-1-yl)propanamide (1) with a biomolecule comprising at least one thiol group; and (ii) subsequently reacting the alkyne group of the intermediate product resulting from step (i) with a fluorine-substituted azide. In another embodiment, the methods may be employed to introduce one or more fluorine atoms onto a biomolecule using Mal-azide bifunctional linker such as compound (2) as the linker. Such methods comprise: (i) reacting the thiol-reactive functional group of compound (2) with a biomolecule comprising at least one thiol group; and (ii) subsequently reacting the azide group of the intermediate product resulting from step (i) with a fluorine-substituted alkyne.

The reaction of the azido or alkyne-reactive terminus of the linker with the fluorine-substituted azide or alkyne may be carried out in any medium that may range from mildly acidic to mild basic conditions. In an embodiment, the reaction may be conducted in a medium having a pH in a range from about 6 to about 9; and in another embodiment, in a pH range from about 7 to about 8. The reaction temperature may be varied from ambient temperature to about 70°C. Reaction time may vary, but generally may be from about 10 minutes to about 60 minutes. In some embodiments, the reaction time varies from about 10 minutes to about 30 minutes. However, longer reaction times may also be employed. The reaction may be carried out in the presence of Cu^{I}, or precursor thereof, including but not limited to Cu^{o}, Cu^{I}, or Cu^{II} salts, in the presence of a reducing reagent, such as but not limited to, sodium ascorbate. Reaction Schemes 3 and 4 show possible approaches to prepare the fluorine labeled bioconjugates (9) and (10).

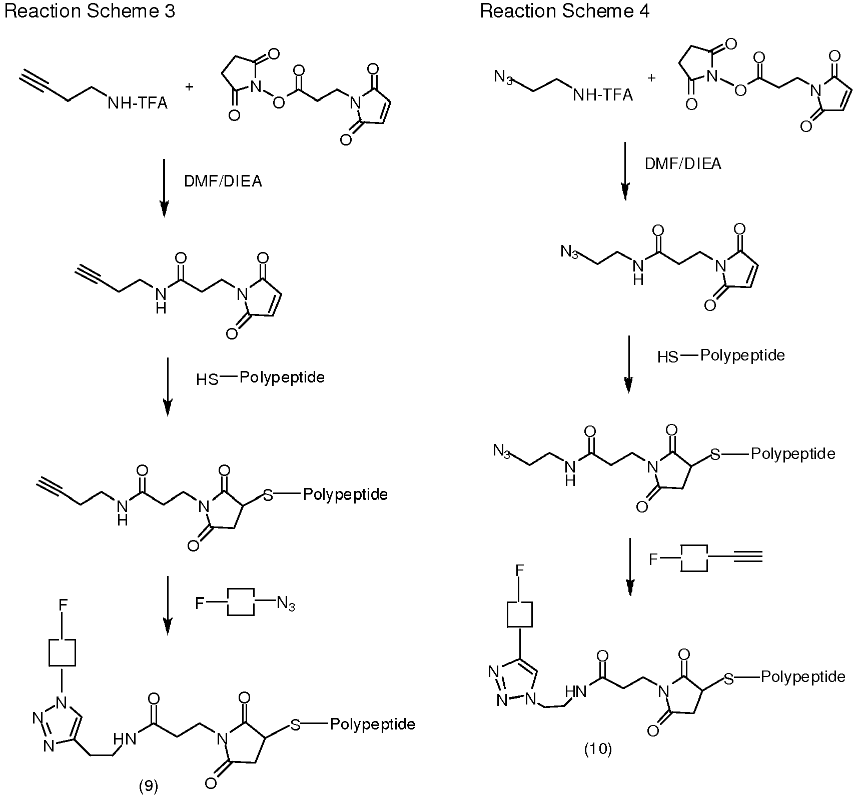

In one approach, the precursor to an alkyne or azide containing linker may be first reacted with a polypeptide comprising a thiol group and the resulting intermediate is transformed to give the desired bioconjugate. Alternatively, the precursor to an alkyne or azide containing linker may be first transformed to give the linker, which may then be reacted with the polypeptide comprising the thiol group to give product. Using the above-described techniques, one may introduce fluorine or radiofluorine atom(s), such as ¹⁸F, onto a biomolecule When a fluorine-substituted azide or alkyne is reacted with a bioconjugate, a fluorine-substituted bioconjugate results. When a radiofluorine-substituted azide or alkyne is reacted with bioconjugate, a radiofluorine-labeled bioconjugate results. Examples of suitable fluorine-substituted azide/alkyne are shown in structures (11)-(14).

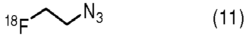

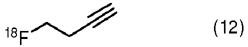

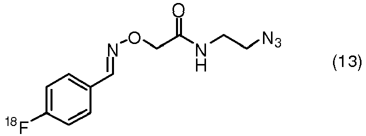

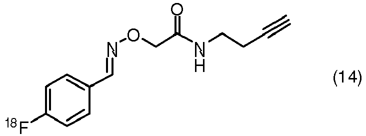

Any of the biomolecules, such as polypeptides, described previously having at least one thiol group may be used for preparing the bioconjugates. Biomolecules such as polypeptide, vectors, scaffold-based proteins and engineered binding proteins having at least one thiol group are especially valuable since such materials have potentially valuable diagnostic and therapeutic value. Thus in an embodiment, bioconjugates may be produced by reacting scaffold-based proteins such as affibodies with a Mal-alkyne bifunctional linker such as N-(but-3-ynyl)-3-(2,5-dioxo-2,5-dihydro-1H-pyrrol-1-yl)propanamide linker (1). In another embodiment, bioconjugates may be produced by reacting scaffold-based proteins such as affibodies with a Mal-azide bifunctional linker such as compound (2). Further, valuable fluorine-labeled bioconjugates may be produced by reacting the bioconjugate with a fluorine-substituted azide or alkyne.

The fluorine-labeled bioconjugates are valuable materials in diagnostic applications. ¹⁸F labeled bioconjugates may be visualized using imaging techniques known in the art, such as for example PET (Positron Emission Tomography) techniques.

### EXAMPLES

Practice of the invention will be still more fully understood from the following examples. The abbreviations used in the Examples section are expanded as follows: "mg": milligrams; "mL": milliliters; "mg/mL": milligrams per milliliter; "mmol": millimoles; "µL": microliter; "KDa": kilodaltons; "MALDI-MS": Matrix Assisted Laser Desorption Ionization Mass Spectrometry; "HPLC": High Pressure Liquid Chromatography; "LC-MS" Liquid Chromatography Mass Spectrometry, "ESI-MS" Electrospray Ionization Mass Spectrometry, "MALDI-TOF MS" Matrix Assisted Laser Desorption Ionization Time Of Flight Mass Spectrometry, "TFA": Trifluoroacetic acid; "DMSO": Dimethylsulfoxide; "DTT": dithiothrietol; "MeCN" acetonitrile, "PBS": phosphate buffered saline, "Mal": Maleimido; "ppm" parts per million, "MWCO: Molecular Weight Cut Off; "cold condition": without radioactive isotope and "radiochemical yield": fraction of activity originally present. Unless otherwise noted, all reagent-grade chemicals were used as received, and Millipore water was used in the preparation of all aqueous solutions.

### Example 1: Preparation of 2-[¹⁸F]Fluoroethylazide (11)

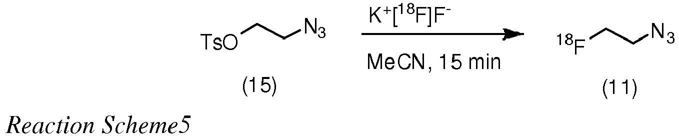

[¹⁸F]-Fluoride was produced by a cyclotron using the ¹⁸O(p,n)¹⁸F nuclear reaction with 19 MeV proton irradiation of an enriched [¹⁸O]H₂O target. After the irradiation, a mixture of Kryptofix® (5 mg, 13.3 µmol), potassium carbonate (1 mg, 7.2 µmol), and MeCN (1 mL) was added to ¹⁸F-water (1 mL). The solvent was removed by heating at 80°C under a stream of nitrogen (100 mL/min). Afterwards, MeCN (0.5 mL) was added and evaporated under heating and nitrogen stream. This procedure was repeated twice. After cooling to room temperature, a solution of toluenesulfonic acid-2-azidoethylester (15) in anhydrous MeCN (0.2 mL) was added. The reaction mixture was stirred for 15 min at 80°C. After addition of MeCN (0.3 mL), 2-[¹⁸F]Fluoroethylazide (11) was distilled at 130°C under a flow of nitrogen (15 mL/min) into a trapping vial containing MeCN (0.1 mL). This compound (11) was collected with a decay-corrected radiochemical yield of 54% (ref. to ¹⁸F-fluoride) and 63% distillation efficiency.

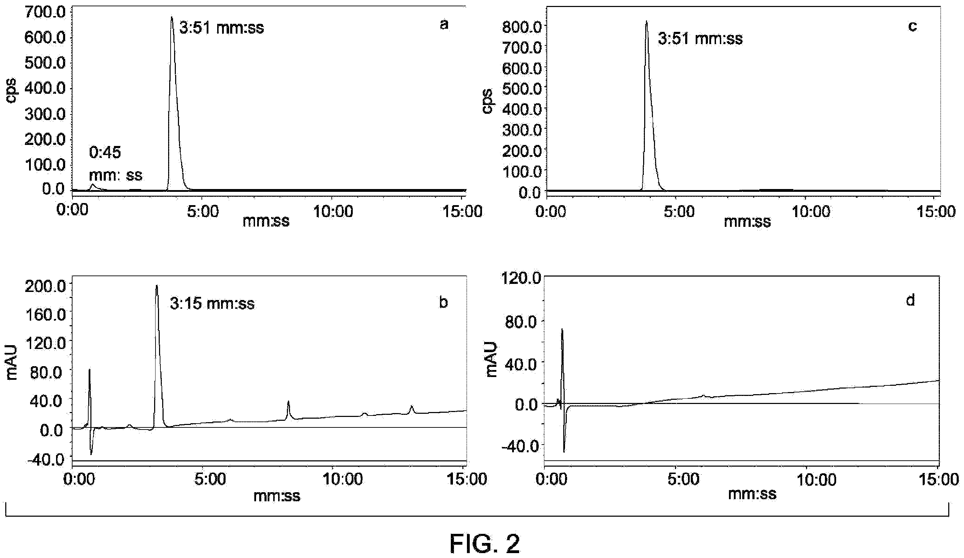

FIG. 2 shows the results of the HPLC analysis of a reaction mixture containing 2-[¹⁸F]Fluoroethylazide (11) using gradient I, UV at 216 nm (a, radioactivity channel, 2-[¹⁸F]Fluoroethylazide (11) at 3:51 min; b, UV channel) and distilled product 2-[¹⁸F]Fluoroethylazide (11) (c, radioactivity channel; d, UV channel).

### Example 2: Concentration Studies Showing Triazole Formation at Different Concentrations of Small Substrate.

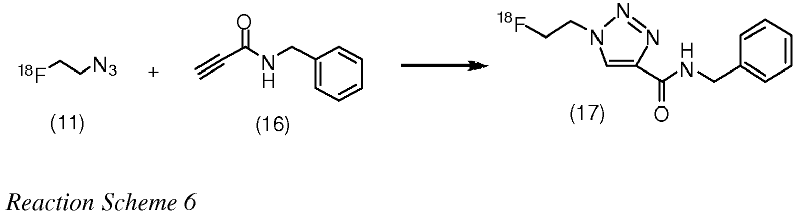

In the concentration study, alkyne model (16) (1.2-0.01 mg, 7.5-0.25 [µmol) was dissolved in DMF (50 [µL) and mixed under nitrogen with an aqueous solution of copper(II) sulfate (25 µl, 2.8 mg, 11.25 µmol), and sodium ascorbate (25 µL, 7.5 mg, 37.5 µmol). After addition of [¹⁸F]2-fluoroethylazide (11) in MeCN (100 µl) and standing for 15 min. at room temperature, the mixture was analyzed by HPLC. The vials were subsequently heated (15 min, 80°C) and again analyzed.

The resulting Triazole product (17) was obtained in quantitative yield at room temperature with a minimum alkyne model (16) concentration of 3.14 mM (100 µg). When the reaction mixture was heated at 80°C the minimum alkyne model (16) concentration was 1.57 mM (50 µg) for a quantitative yield of resulting Triazole (17).

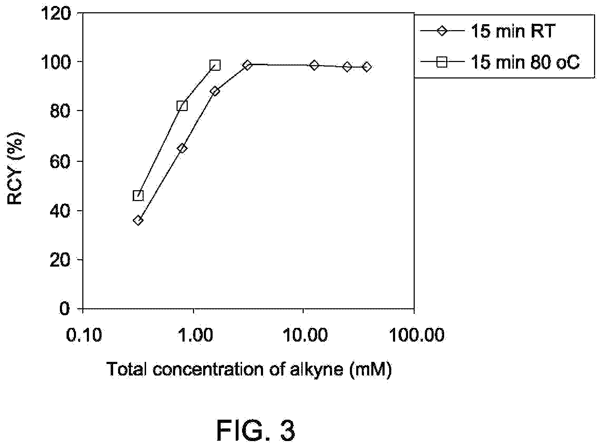

Fig. 3 shows the HPLC plot of radiochemical yield of triazole product (17) formed at different alkyne (16) concentrations and temperatures using model compound (16) and 2-[18F]Fluoroethylazide (11)

### Example 3: Triazole Formation on Peptide

Preparation of [¹⁸F](S)-6-Amino-2-(2-{(S)-2-[2-((S)-6-amino-2-{[4-(2-fluoro-ethyl)-[1,2,3]triazole-1-carbonyl]-amino}-hexanoylamino)-acetylamino]-3-phenyl-propionylamino}-acetylamino)-hexanoic acid (19)

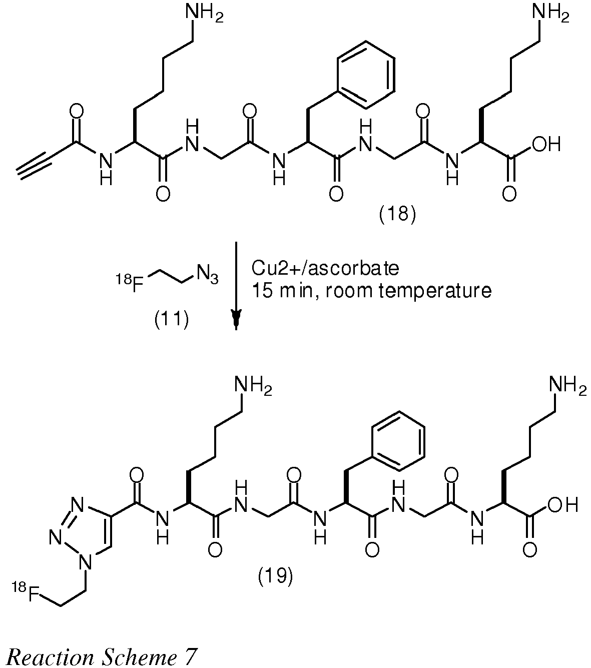

To a solution of copper(II) sulphate pentahydrate (4.3 mg, 17 µmol) in water (50 µL), was added sodium ascorbate (3.4 mg, 17 µmol) in water (50 µL) under nitrogen. After approximately one minute at room temperature, a solution of alkyne peptide precursor (18) was added (2 mg, 3.4 µmol, in sodium phosphate buffer, pH 6.0, 0.5 M, 50 µL), followed by a solution of 2-[¹⁸F]Fluoroethylazide (11) (24-32 MBq) in MeCN(50 µL). The mixture was kept at room temperature for 15 minutes and diluted with HPLC mobile phase A (water, 0.1 % TFA) (0.3 mL). The labeled peptide (19) was isolated using semi-preparative HPLC (UV at 216 nm, Rt = 6:24).

### Example 4: Synthesis of Mal-alkyne Bifunctional Linker (1)

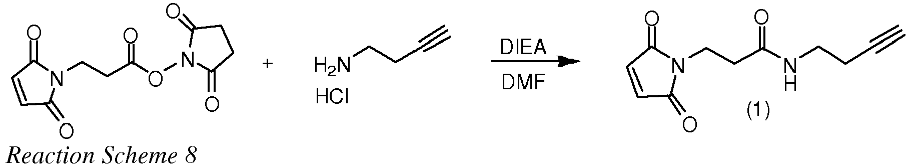

*N*-[β-Maleimidopropyloxy]succinimide ester (50 mg, 1.25 equiv) was dissolved in 1.0 mL of dry DMF. 3-Butyn-1-amine hydrochloride (16 mg, 1.0 equiv) was dissolved in 0.5 mL of dry dimethylformamide (DMF) and 26 µL of diisopropylethylamine (DIEA). This amine solution was added drop-wise to the succinimide ester while keeping the ester solution in an ice bath. The mixture was stirred at 0°C for 10 min. The solution was warmed up to room temperature and stirred for 18 h. The solvents were evaporated under vacuum and the residue was dissolved in 5 mL CH₂Cl₂. The organic solution was extracted with brine (3 x 5 mL) and dried over MgSO₄. The solvent was removed under reduced pressure and the crude material was purified using flash chromatography (silica, MeOH/CH₂Cl₂). The product (1) was purified from grease by dissolving the sample in a minimum amount of CH₂Cl₂ (ca. 2 mL), followed by three washes with hexanes. The product (1) precipitated as a fluffy white solid. Characterization of the product was achieved using ¹H-NMR. Yield: 8.2 mg (25%). ¹H-NMR (500 MHz, CDCl₃) : δ 2.02 (s, 1), 2.41 (t, J = 5Hz, 2), 2.57 (t, J = 5 Hz, 2), 3.42 (td, J= 5 Hz, 2), 3.88 (t, J= 5 Hz), 5.90 (bs, 1), 6.73 (s, 2).

### Example 5: Preparation of 2-Fluoroethyltriazole-Maleimide (Reference Compound) (21)

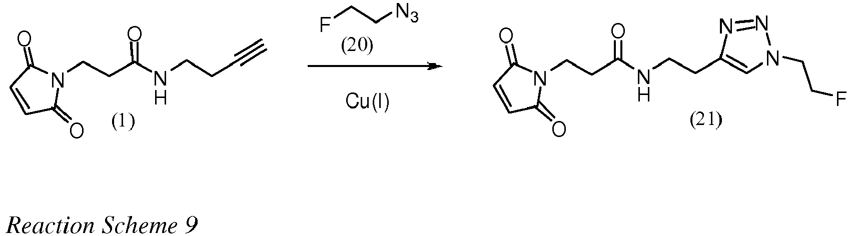

A mixture of sodium phosphate buffer (20 µl, 250 mM, pH 6.0), DMF (50 µl) and copper powder (100 mg, -40 mesh, Aldrich Cat. No. 26,608-6) was purged with nitrogen gas (10 ml/min) for 5 minutes. After addition of 2-fluoroethylazide (20) (10 µmol) in DMF (9.2 µl, prepared as described by Glaser and Årstad 2007), and 3-(N-maleimidyl)-N-(3-propargyl) propionamide (1) (2.1 mg, 9.5 µmol) in DMF (20 µl), the vessel was stirred at room temperature for 1 hour. The solvent was removed under reduced pressure (1 mbar, 80°C). Purification by preparative HPLC gave the fluoroethyltriazole (21) compound (1.2 mg, 41%). HR ESI-MS: for C₁₃H₁₆N₅O₃F (Calculated. m/z = 310.1310, Found m/z = 310.1314).

Purification by preparative HPLC (gradient: 5-80 % solvent B over 15 min where A= H₂O /0.1 % TFA and B= MeCN /0.1 % TFA, flow rate: 15 mL/min; column: Luna 5 µm C18(2) (Phenomenex), 75x30 mm, detection: UV 216 nm.

### Example 6: Affibody Alkynylation and Reaction with Mal-alkyne Bifunctional Linker

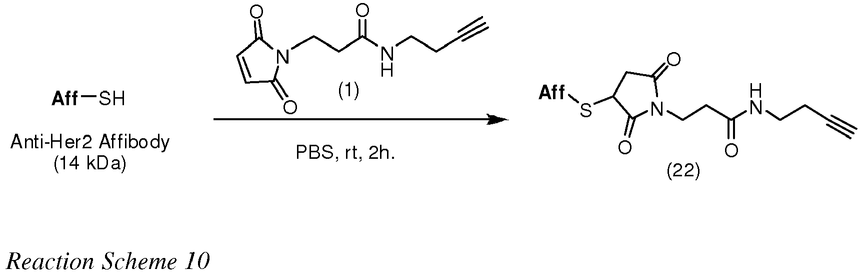

Anti-Her2 affibody, 14 kDa (1 mg as a lyophilized powder) was dissolved in 460 µL of phosphate buffered saline (PBS). The pH of the solution was maintained at 7.4. To this solution was added 40 µL of a 0.5 M solution of dithiothreitol (DTT) dissolved in PBS; the pH was maintained at 7.4. The resulting mixture was stirred at room temperature for 2h. The reduced affibody was purified via gel filtration. To this pure fraction of reduced affibody was added a 12 µL aliquot of a solution of the Mal-alkyne Bifunctional Linker (1) (0.15 M in DMSO). This reaction mixture was incubated at room temperature for 2h with moderate stirring. The reaction mixture was purified using gel filtration followed by Centrifugal Ultrafiltration (Amicon), MWCO 5 kDa (Millipore) to obtain purified Alkynylated affibody (22).

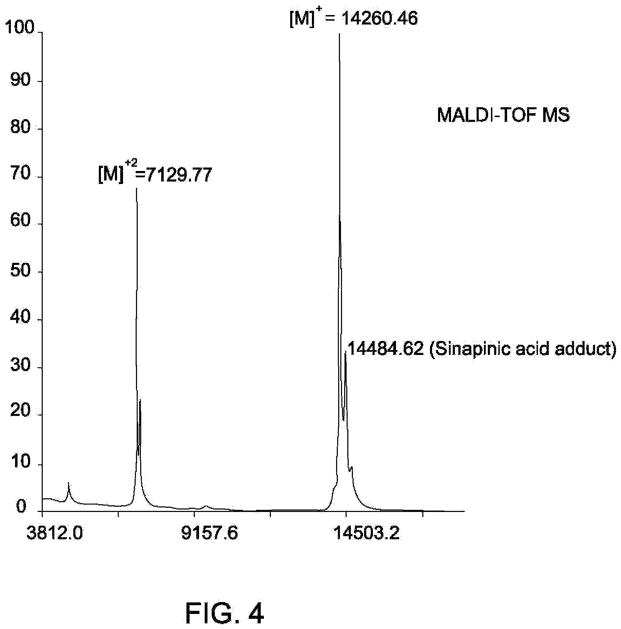

Characterization of the resulting purified Alkynylated affibody (22) was achieved using (MALDI-TOF-MS) and HPLC followed by LC(ESI)-MS. Yield (%): 78; MS: (MH⁺ calculated: 14260, found: 14260) FIG. 4 shows the MALDI-TOF MS data of Alkynylated affibody (22).

Standard conditions for HPLC analysis of Alkynylated affibody (22): Grace Vydac Protein C4 column was used for HPLC analysis. Solvent A: 100% H₂O, 0.1% TFA, Solvent B: 100% MeCN, 0.1% TFA; detection: UV 216 and 280 nm; flow rate: 1 mL/min. The solvent concentration was changed with time as shown in Table 1 below.

**Table 1**

| Time | % A | % B |
|---|---|---|
| 0 | 100 | 0 |
| 4 | 80 | 20 |
| 16 | 40 | 60 |
| 20 | 0 | 100 |

The typical elution time for the Alkynylated affibody (22) was approximately 11 minutes.

### Example 7: Triazole Formation on Bioconjugate Under Cold Conditions.

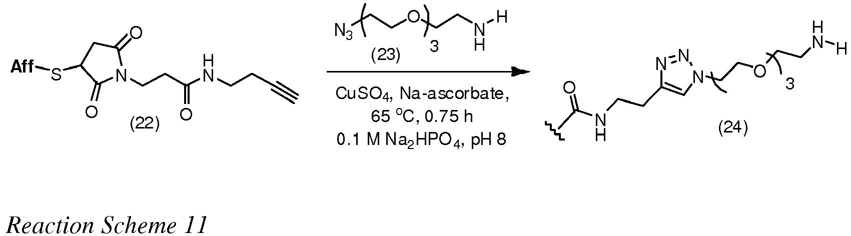

Labeling of Alkynylated-affibody (22) using Azido-PEG (23). To 36 µL of an aqueous solution of sodium phosphate (100 mM, pH 8) was added a 3 µL aliquot of a stock of Alkynylated affibody (22) solution (0.6 mM, ∼8 mg/mL in phosphate buffered saline, at pH 7.4). To this solution µL aliquot of a freshly prepared 18 mM solution of 11-Azido-3,6,9-trioxaundecan-1-amine (Azido-PEG) (23) (diluted in 100 mM sodium phosphate, at pH 8) was added. To this solution was then added a 5 µL aliquot of a freshly prepared 10 mM (2 mg/mL) solution of sodium ascorbate (in 100 mM sodium phosphate, pH 8). Finally, a 5 µL aliquot of a freshly prepared 10 mM (1.6 mg/mL) suspension of copper (II) sulfate (in 100 mM sodium phosphate, pH 8) was added to the reaction mixture. These additions thus yielded the following final concentrations for reagents and substrate- Alkynylated affibody (22) (36 µM), the Azido PEG (23) (360 µM), sodium ascorbate (1 mM), and CuSO4 (1 mM).

The resulting solution was mixed at 65°C with moderate agitation for 0.75 h. The reaction mixture was then immediately diluted to 4 mL in pure water (MilliQ) and concentrated to <100 µL using an Amicon Centrifugal Ultrafiltration unit, MWCO 5kDa (Millipore). This water dilution-concentration sequence was repeated two additional times in order to remove excess reagents. Characterization of the resulting purified conjugate (24) was achieved using MALDI-TOF-MS and High Performance Liquid Chromatography followed by LC(ESI)-MS. Yield (%): 42. MS: (MH⁺ calculated: 14478, found: 14492).

### Example 8: Labeling of Alkynylated-affibody (22) Using Cy5-labeled Azido PEG (25a)

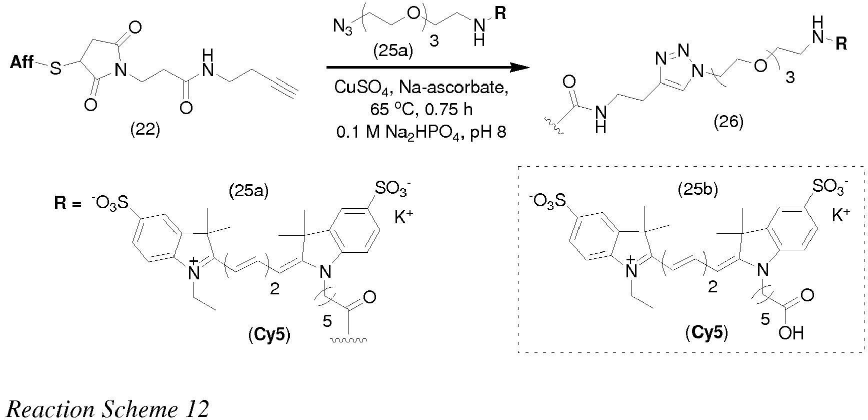

To 36 µL of an aqueous solution of sodium phosphate (100 mM, pH 8) was added a 3 µL aliquot of a stock of Alkynylated affibody (22) solution (0.6 mM, ∼8 mg/mL in phosphate buffered saline, pH 7.4). Next was added a 1 µL aliquot of a freshly prepared 18 mM solution of Cy5-labeled azido-PEG (25a) (diluted in 100 mM sodium phosphate, pH 8). To this solution, a 5 µL aliquot of a freshly prepared 10 mM (2 mg/mL) solution of sodium ascorbate (in 100 mM sodium phosphate, pH 8) was then added. Finally, a 5 µL aliquot of a freshly prepared 10 mM (1.6 mg/mL) suspension of copper (II) sulfate (in 100 mM sodium phosphate, pH 8) was added to the reaction mixture. These additions yielded the following final concentrations for reagents and substrate: Alkynylated affibody (22) (36 µM), Cy5-labeled azido-PEG (25a) (360 µM), sodium ascorbate (1 mM), and CuSO4 (1 mM).

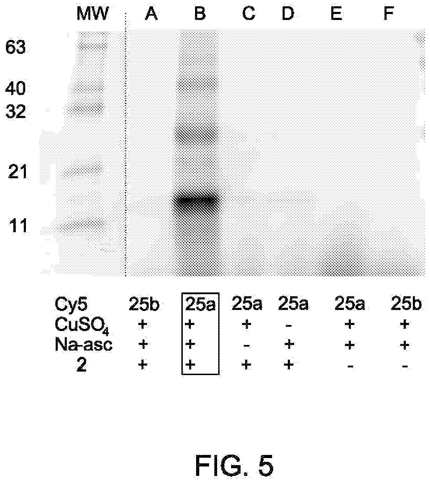

The resulting solution was mixed at 65°C with moderate agitation for 0.75 hours. The reaction mixture was then immediately diluted to 4 mL in pure water (MilliQ) and concentrated to <100 µL using an Amicon Centrifugal Ultrafiltration unit, MWCO 5kDa (Millipore). This water dilution-concentration sequence was repeated two additional times in order to remove excess reagents. Characterization of the resulting purified conjugate (26) was achieved by HPLC and SDS-PAGE protein gel monitoring the dye's fluorescence emission (FIG. 5).

### Example 9: Triazole Formation on Alkynylated affibody (22) Under Hot Conditions

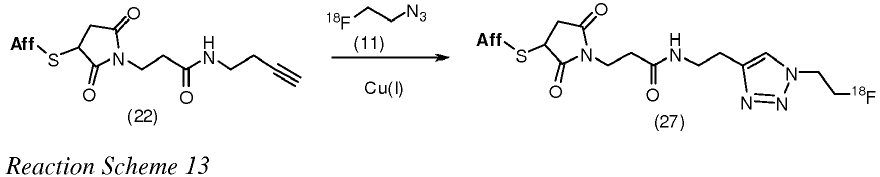

Labeling of Anti-Her2 affibody (22) with ¹⁸F. An aqueous solution of copper(II) sulfate (5 µL, 0.0249 mg, 0.1 µmol) was mixed with sodium ascorbate (0.198 mg, 1 µmol) in sodium phosphate buffer (5 µL, 100 mM, pH 8.0) and alkynylated affibody (22) (50 µg, 3.57 nmol) in PBS (5 µL). After addition of 2-[¹⁸F]fluoroethylazide (11) (264 µCi, 9.8 MBq, prepared as described by Glaser, M., and Årstad, E. (2007), "Click labeling' with 2-[18F]fluoroethylazide for Positron Emission Tomography. Bioconj. Chem. 18, 989-993, which is incorporated herein by reference]. in MeCN (20 µl), the mixture was heated at 60°C for 30 min. Labeling with 2-[18F]fluoroethylazide (11) HPLC analysis of the reaction mixture showed a radioactivity peak co-eluting with the affibody (27) UV signal.

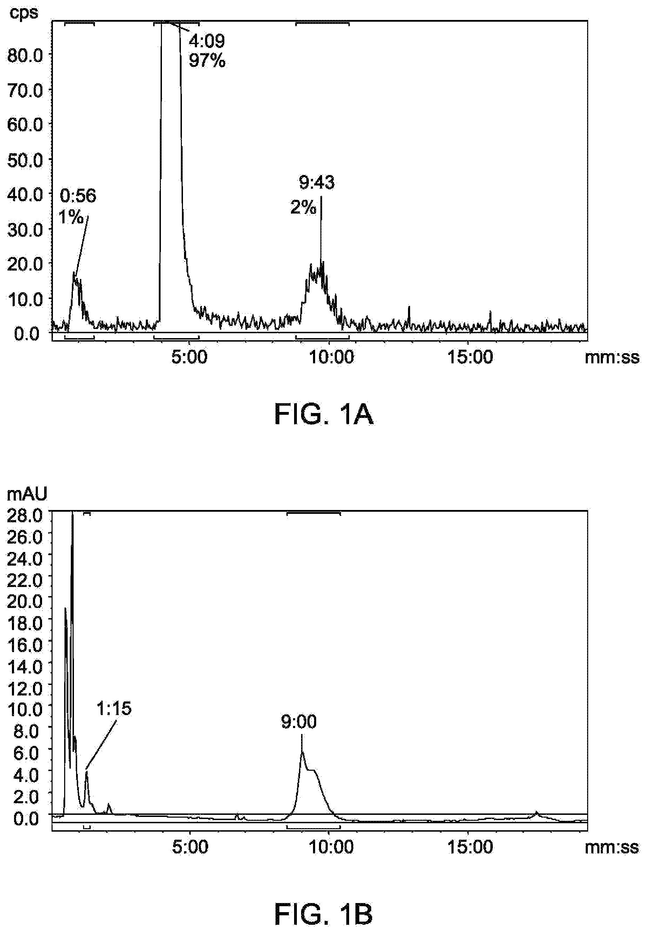

Purification by preparative HPLC (gradient: 5-80 % solvent B over 15 min where A= H₂O /0.1 % TFA and B= MeCN /0.1 % TFA, flow rate: 1 mL/min; column: Luna 3 µm C18(2) (Phenomenex), 50x4.6 mm, detection: UV 280 nm. FIG. 1 shows HPLC analysis of a reaction mixture of the non-optimized system showing ¹⁸F click labeled Anti-Her2 affibody (27) (a, radioactivity channel; b, UV channel at 280 nm).

## Claims

1. A method for introducing a fluorine atom onto a biomolecule, comprising:
(i) providing a linker including a thiol-reactive terminus and an azido or an alkyne-reactive terminus;
(ii) reacting the thiol-reactive terminus of the linker with the biomolecule including at least one thiol group or a derivative of the thiol group which can be activated so as to generate the free thiol group; and
(iii) subsequently reacting the azido or the alkyne-reactive terminus of the linker with a fluorine-substituted azide or alkyne group;
wherein the linker is: 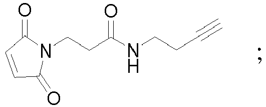 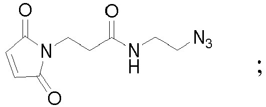 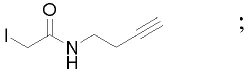 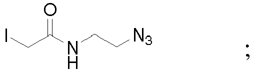 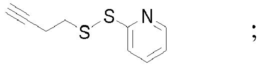 or

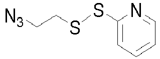

2. The method of claim 1, wherein the biomolecule has more than 50 amino acid residues.

3. The method of claim 1 or 2, wherein the biomolecule is an affibody.

4. The method of any one of claims 1 to 3, wherein the at least one thiol group includes a cysteine residue or a thiol-containing non-natural amino acid.

5. The method of any one of claims 1 to 4, wherein the reactive derivative of the biomolecule is produced by treating the biomolecule with a reducing agent.

6. The method of any one of claims 1 to 5, wherein the fluorine-substituted azide or alkyne comprises a ¹⁸F substituted azide or alkyne or a ¹⁹F substituted azide or alkyne.

7. A bioconjugate comprising:
(i) a biomolecule comprising at least one thiol group; and
(ii) a linker conjugated to the thiol group of said biomolecule;
wherein the linker is: 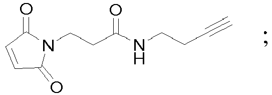 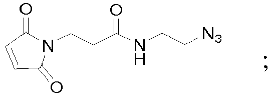 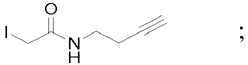 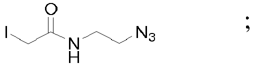 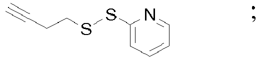 or 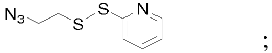 and is prepared by a method comprising reacting an amine compound comprising an azido or alkyne-reactive functional group with a carboxylic acid or an activated ester comprising a thiol-reactive functional group.

8. The bioconjugate of claim 7, wherein the activated ester comprises a structure: L-M-COR²; wherein L comprises a thiol-reactive functional group, or a protected derivative thereof; M is a divalent linking unit; and R² is OH or an activating group.

9. The bioconjugate of claim 7, wherein the amine compound comprises a structure: G-J-R¹; wherein G is a amine, J is a divalent linking unit, and R¹ is an azido or an alkyne-reactive functional group.

10. The bioconjugate of claim 7, wherein the biomolecule is an affibody.

11. A linker selected from the group consisting of: 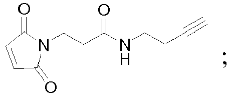 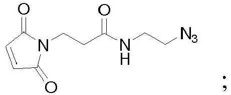 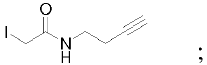 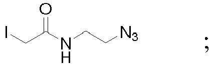 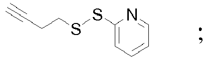 and

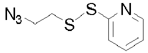

## Patentansprüche

1. Verfahren zum Einbringen eines Fluoratoms auf ein Biomolekül, umfassend:
(i) Bereitstellen eines Linkers, der einen Thiol-reaktiven Terminus und einen Azido- oder einen Alkyn-reaktiven Terminus einschließt;
(ii) Reagieren des Thiol-reaktiven Terminus des Linkers mit dem Biomolekül, das mindestens eine Thiolgruppe oder ein Derivat der Thiolgruppe aufweist, die aktiviert werden können, um die freie Thiolgruppe zu bilden, und
(iii) anschließend Reagieren des Azido - oder des Alkyn-reaktiven Terminus des Linkers mit einer fluorsubsituierten Azid- oder Alkyngruppe,
wobei der Linker wie folgt ist: 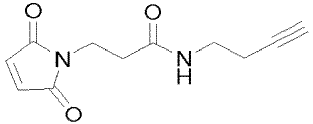 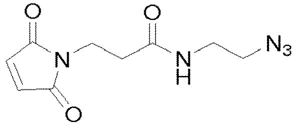 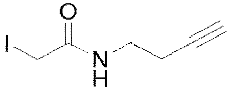 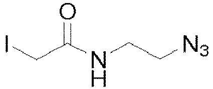 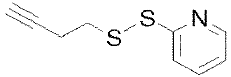 oder

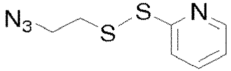

2. Verfahren nach Anspruch 1, wobei das Biomolekül mehr als 50 Aminosäurereste hat.

3. Verfahren nach Anspruch 1 oder 2, wobei das Biomolekül ein Affibody (künstliches, vom Protein A aus dem Bakterium "Staphylococcus aureus" abgeleitetes Peptid) ist.

4. Verfahren nach einem der Ansprüche 1 bis 3, wobei die mindestens eine Thiolgruppe einen Cysteinrest oder eine Thiol-haltige, nicht natürliche Aminosäure enthält.

5. Verfahren nach einem der Ansprüche 1 bis 4, wobei das reaktive Derivat des Biomoleküls durch Behandeln des Biomoleküls mit einem Reduktionsmittel hergestellt wird.

6. Verfahren nach einem der Ansprüche 1 bis 5, wobei das Fluor-subsituierte Azid oder Alkyn ein ¹⁸F-substituiertes Azid oder Alkyn oder ein ¹⁹F-subsistuiertes Azid oder Alkyn ist.

7. Ein Biokonjugat, umfassend:
(i) ein Biomolekül, das mindestens eine Thiolgruppe umfasst, und
(ii) einen Linker, der mit der Thiolgruppe des Biomoleküls konjugiert ist,
wobei der Linker folgender ist: 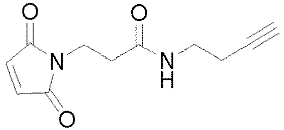 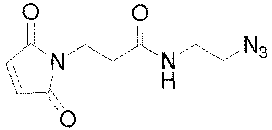 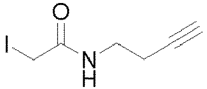 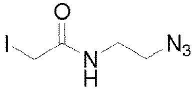 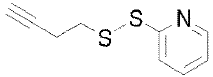 oder 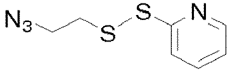 und nach einem Verfahren hergestellt ist, welches das Reagieren einer Aminverbindung umfasst, die eine Azido- oder Alkyn-reaktive Funktionsgruppe mit einer Karboxylsäure oder einem aktivierten Ester umfasst, der eine Thiol-reaktive Funktionsgruppe aufweist.

8. Das Biokonjugat nach Anspruch 7, wobei der aktivierte Ester eine Struktur: L-M-COR² aufweist, wobei L eine Thiol-reaktive Funktionsgruppe oder ein geschütztes Derivat derselben aufweist, M eine divalente Verlinkungseinheit ist und R² OH oder eine Aktivierungsgruppe ist.

9. Biokonjugat nach Anspruch 7, wobei die Aminverbindung eine Struktur: G-J-R¹ umfasst, wobei G ein Amin ist, J eine divalente Verlinkungseinheit ist und R¹ eine Azido- oder eine Alkyn-reaktive Funktionsgruppe ist.

10. Biokonjugat nach Anspruch 7, wobei das Biomolekül ein Affibody ist.

11. Ein Linker, der aus der Gruppe ausgewählt ist, bestehend aus: 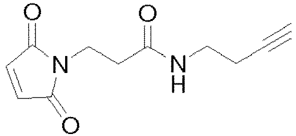 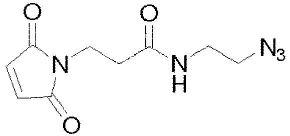 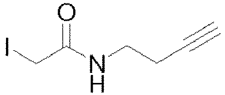 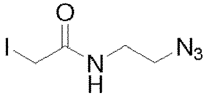 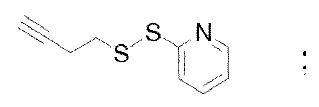 und

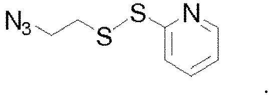

## Revendications

1. Procédé d'introduction d'un atome de fluor sur une biomolécule, comprenant :
(i) la fourniture d'un lieur comprenant une extrémité réactive au thiol et une extrémité réactive à un azoture ou à un alcyne ;
(ii) la réaction de l'extrémité réactive au thiol du lieur avec la biomolécule comprenant au moins un groupement thiol ou un dérivé du groupement thiol qui peut être activé de manière à générer le groupement thiol libre ; et
(iii) ensuite la réaction de l'extrémité réactive à l'azoture ou à l'alcyne du lieur avec un groupement azoture ou alcyne substitué par du fluor ;
dans lequel le lieur est l'un des suivants : 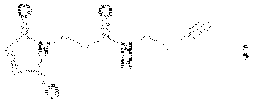 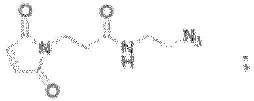 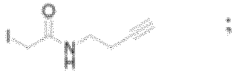 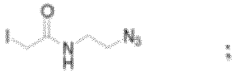 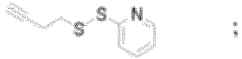 ou

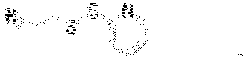

2. Procédé selon la revendication 1, dans lequel la biomolécule a plus de 50 résidus d'acides aminés.

3. Procédé selon la revendication 1 ou 2, dans lequel la biomolécule est un affibody.

4. Procédé selon l'une quelconque des revendications 1 à 3, dans lequel le au moins un groupement thiol comprend un résidu de cystéine ou un acide aminé non naturel contenant du thiol.

5. Procédé selon l'une quelconque des revendications 1 à 4, dans lequel le dérivé réactif de la biomolécule est produit en traitant la biomolécule avec un agent réducteur.

6. Procédé selon l'une quelconque des revendications 1 à 5, dans lequel l'azoture ou l'alcyne substitué par du fluor comprend un azoture ou un alcyne substitué par ¹⁸F ou un azoture ou un alcyne substitué par ¹⁹F.

7. Bioconjugué comprenant :
(i) une biomolécule comprenant au moins un groupement thiol ; et
(ii) un lieur conjugué au groupement thiol de ladite biomolécule ;
dans lequel le lieur est l'un des suivants : 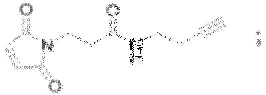 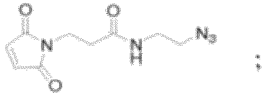 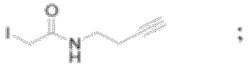 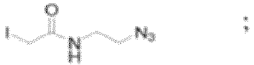 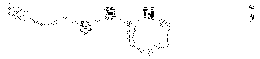 ou 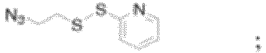 et est préparé par un procédé comprenant la réaction d'un composé aminé comprenant un groupement fonctionnel réactif à un azoture ou à un alcyne avec un acide carboxylique ou un ester activé comprenant un groupement fonctionnel réactif au thiol.

8. Bioconjugué selon la revendication 7, dans lequel l'ester activé comprend une structure L-M-COR²; dans lequel L comprend un groupement fonctionnel réactif au thiol ou un dérivé protégé de celui-ci ; M est une unité de liaison bivalente ; et R² est OH ou un groupe d'activation.

9. Bioconjugué selon la revendication 7, dans lequel le composé aminé comprend une structure G-J-R¹ ; dans lequel G est une amine, J est une unité de liaison bivalente et R¹ est un groupement fonctionnel réactif à un azoture ou à un alcyne.

10. Bioconjugué selon la revendication 7, dans lequel la biomolécule est un affibody.

11. Lieur choisi dans le groupe constitué des suivants : 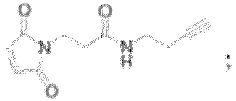 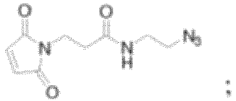 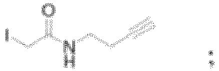 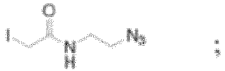 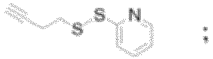 et
